(19) **Europäisches Patentamt**
**European Patent Office**
**Office européen des brevets**

(11) Veröffentlichungsnummer : **0 483 518 B1**

(12) **EUROPÄISCHE PATENTSCHRIFT**

(45) Veröffentlichungstag der Patentschrift :
**27.04.94 Patentblatt 94/17**

(51) Int. Cl.$^5$ : **C07C 51/47**, C07C 51/573,
C07C 51/12

(21) Anmeldenummer : **91116520.7**

(22) Anmeldetag : **27.09.91**

(54) **Verfahren zur Entfernung metallischer Korrosionsprodukte aus wasserfrei betriebenen Carbonylierungsreaktionen.**

(30) Priorität : **30.10.90 DE 4034501**

(43) Veröffentlichungstag der Anmeldung :
**06.05.92 Patentblatt 92/19**

(45) Bekanntmachung des Hinweises auf die
Patenterteilung :
**27.04.94 Patentblatt 94/17**

(84) Benannte Vertragsstaaten :
**AT BE CH DE ES FR GB IT LI NL**

(56) Entgegenhaltungen :
**EP-A- 0 265 140**
**GB-A- 2 020 257**
**US-A- 4 007 130**

(73) Patentinhaber : **HOECHST
AKTIENGESELLSCHAFT
D-65926 Frankfurt (DE)**

(72) Erfinder : **Erpenbach, Heinz, Dr.
Oberbuschweg 22
W-5000 Köln (DE)**
Erfinder : **Gradl, Reinhard, Dr.
Heimbacher Weg 27
W-5042 Erftstadt (DE)**
Erfinder : **Jägers, Erhard, Dr.
Hemberger Strasse 75
W-5303 Bornheim (DE)**
Erfinder : **Seidel, Andreas, Dr.
Luxemburger Strasse 469
W-5000 Köln (DE)**
Erfinder : **Prinz, Peter
Von-Geyr-Ring 104
W-5030 Hürth (DE)**

## Beschreibung

Die Erfindung betrifft ein Verfahren zur Entfernung metallischer Korrosionsprodukte aus wasserfrei betriebenen Carbonylierungsreaktionen, bei denen Methylacetat und/oder Methanol und/oder Dimethylether an einem Edelmetallkatalysatorsystem, das aus einem Edelmetall der Gruppe VIII des Periodensystems der Elemente, einem Co-Katalysator wie Iodid oder Bromid, insbesondere Methyliodid, und einem Promotor wie Organophosphonium- oder Organoammonium-Salz sowie gegebenenfalls Lithium-Salz besteht, zu Essigsäure und/oder Essigsäureanhydrid umgesetzt werden.

Aus wirtschaftlichen Gründen steigt der Produktionsanteil an Essigsäure und Essigsäureanhydrid, welcher durch Carbonylierung hergestellt wird, laufend an. Hierbei ist es wichtig, die teueren Edelmetalle sowie Organophosphonium- und Organoammonium-Salze verlustfrei anzuwenden.

Es hat sich herausgestellt, daß in der im Kreislauf geführten Reaktionslösung metallische Korrosionsprodukte wie Eisen, Nickel, Chrom und Molybdän angereichert werden. Hierdurch tritt eine Verschlechterung der Wirksamkeit des Katalysatorsystems auf.

Dem Fachmann ist deshalb die Aufgabe gestellt, die metallischen Korrosionsprodukte aus der Reaktionslösung ohne Einbuße an Katalysatorbestandteilen zu entfernen.

In der US-A-4,007,130 ist ein Verfahren zur Abtrennung metallischer Korrosionsprodukte mittels Ionenaustauscher beschrieben. Die metallischen Korrosionsprodukte werden am Ionenaustauscher adsorbiert, die Edelmetalle verbleiben im Eluat und können direkt wieder als Katalysatorlösung rückgeführt werden. Katalysatorrestmengen werden mit Wasser in die Katalysatorlösung gespült, bevor der mit metallischen Korrosionsprodukten beladene Ionenaustauscher mit Mineralsäuren regeneriert wird.

Es hat sich gezeigt, daß dieses Verfahren auf wasserfreie Carbonylierungen, die als Promotor Organophosphonium- oder Organoammoniumiodid enthalten, nicht anwendbar ist, da die Promotoren ebenfalls am Ionenaustauscher adsorbiert werden. Zum wirtschaftlichen Betrieb der Carbonylierung ist aber die Rückgewinnung sowohl der Edelmetalle als auch des Promotors notwendig. Eine Eluierung des Katalysators mit Wasser, wie sie in der US-A-4,007,130 vorgesehen ist, ist nicht durchführbar, da zur Vermeidung einer vergrößerten Korrosion im Carbonylierungsverfahren unter wasserfreien Bedingungen gearbeitet werden muß (DE-A-3 429 179 ≈ CA-A-1 258 469).

Mit dem Verfahren der Erfindung können die im Stand der Technik aufgezeigten Nachteile vermieden werden. Mittels eines Ionenaustausch-Verfahrens werden die metallischen Korrosionsprodukte so ausgeschleust, daß einer wasserfrei betriebenen Carbonylierung sowohl die Edelmetalle als auch die Organophosphonium- oder Organoammonium-Verbindungen ohne weitere Aufarbeitung als Katalysatorsystem im Kreislauf der Reaktionslösung rückgeführt werden können.

Im einzelnen ist die Erfindung dadurch gekennzeichnet, daß man

a) die mit metallischen Korrosionsprodukten verunreinigte, das Katalysatorsystem enthaltende Reaktionslösung mit einem Ionenaustauscher kontaktiert;

b) diese so kontaktierte Reaktionslösung von dem Ionenaustauscher trennt;

c) den am Ionenaustauscher adsorbierten Promotor vor einer Regenerierung mit Essigsäure und/oder Essigsäureanhydrid desorbiert;

d) das in Schritt c) erhaltene Eluat mit der in Schritt b) abgetrennten Reaktionslösung vereinigt und gemeinsam in die Carbonylierungsreaktion rückführt;

e) den aus Schritt c) erhaltenen Ionenaustauscher mit starker Mineralsäure regeneriert;

f) den so regenerierten Ionenaustauscher mit Essigsäure und/oder Essigsäureanhydrid vor dem Einsatz in Schritt a) wasserfrei wäscht.

Das Verfahren der Erfindung kann weiterhin bevorzugt und wahlweise dadurch gekennzeichnet sein, daß man

aa) einen starksauren makroporösen Ionenaustauscher einsetzt, der aus einem Sulfonsäuregruppen enthaltenden Styrol/Divinylbenzol-Polymerisat besteht;

bb) den Schritt a) bei Temperaturen zwischen 20 und 120 °C, insbesondere 75 und 100 °C, und Drucken von 1 bis 10 bar durchführt;

cc) die verunreinigte Reaktionslösung durch ein festes Ionenaustauscherbett leitet;

dd) die verunreinigte Reaktionslösung in einem Fließbett mit dem Ionenaustauscher behandelt;

ee) die verunreinigte Reaktionslösung in einem Rührbehälter chargenweise mit dem Ionenaustauscher behandelt;

ff) im Schritt e) als starke Mineralsäure 0,5 bis 5, insbesondere 1 bis 3 molare Jodwasserstoffsäure, Schwefelsäure oder Salzsäure einsetzt.

Als Edelmetall enthält das Katalysatorsystem insbesondere Rhodium- und Iridium-Verbindungen. Als Co-Katalysator können neben Methyliodid auch noch elementares Jod oder Jodwasserstoff genutzt werden.

Als Promotor werden insbesondere quartäre Phosphoniumsalze wie Methyltributylphosphonium-, Methyltriphenylphosphonium-, Tetrabutylphosphonium- oder Dimethyldibutylphosphoniumiodid sowie N,N-Dimethylimidazolium-, N-Methyl-3-picolinium- oder Methyl-chinoliniumiodid zugegeben.

In der Carbonylierungsreaktion hat sich ein Molverhältnis Edelmetall zu Methyliodid zu Promotor zu Methylacetat/Methanol/Dimethylether-Gemisch von 1 : (10-300) : (2-100) : (10-1000) bei einer Edelmetallkonzentration von 0,005 bis 0,05 mol/l bewährt.

Als starksaure makroporöse Ionenaustauscher können ®Amberlyst 15 (Firma Rohm and Haas Deutschland GmbH, 6000 Frankfurt 1), ®Lewatit SPC 112 sowie SPC 118 (Firma Bayer AG, 5090 Leverkusen-Bayerwerk) verwendet werden.

Vor der Entfernung der metallischen Korrosionsprodukte aus der Reaktionslösung wird ein Teil der Carbonylierungsprodukte (Essigsäure, Essigsäureanhydrid) aus der Reaktionslösung destillativ abgetrennt. Der dann mit dem Ionenaustauscher kontaktierte Destillationssumpf enthält

30 bis 65 Gew.-%      Promotor (Methyltributylphosphoniumiodid)
25 bis 60 Gew.-%      organische Verbindung (Essigsäure, Essigsäureanhydrid, Ethylidendiacetat)
2 bis 15 Gew.-%       Rhodiumcarbonylkomplex

$$[Rh(CO)_2I_2] [CH_3P(C_4H_9)_3]$$

```
0,5  bis  1,0 Gew.-% Fe ⎫
0,05 bis  0,5 Gew.-% Cr ⎪  in Form löslicher
0,03 bis  0,3 Gew.-% Ni ⎬  Verbindungen
0,01 bis  0,1 Gew.-% Mo ⎭
```

Beispiel 1

120 ml ®Lewatit SPC 118 in der $H^+$-Form wurden mit einem Essigsäure/Essigsäureanhydrid-Gemisch (1 : 1 Volumenteile) überschichtet und zum Quellen über Nacht gelagert. Zur Adsorption wurde der Ionenaustauscher mitsamt der überstehenden Flüssigkeit in eine mantelbeheizte Säule (20 mm Ø und 400 mm Länge) gepackt und auf 80 °C erwärmt. Bei dieser Temperatur wurden 700 g verunreinigte und destillativ angereicherte Reaktionslösung mit einer Fließgeschwindigkeit von 20 ml/min über den Ionenaustauscher geleitet. Die Reaktionslösung bestand aus: 31,01 Gew.-% Essigsäure, 11,00 Gew.-% Essigsäureanhydrid, 29,58 Gew.-% Methyltributylphosphoniumiodid, 22,63 Gew.-% nicht bestimmte organische Phase und 5,78 Gew.-% Metallsalze inclusive Rhodiumkomplex.

Der mit den Korrosionsprodukten beladene Ionenaustauscher wurde nach vollständigem Ablassen der Katalysatorlösung mit 500 ml Essigsäure-Essigsäureanhydrid-Gemisch (19 : 1 Volumenteile) bei 80 °C behandelt. Das Eluat wurde mit der gereinigten Reaktionslösung vereinigt. Der beladene und mit Essigsäure/Essigsäureanhydrid behandelte Ionenaustauscher wurde mit 1000 g einer 1,5 molaren Salzsäure bei 23 °C regeneriert. Im dabei anfallenden Eluat wurde die Menge der desorbierten Metalle ermittelt.

In der Tabelle 1 sind die Ad- und Desorptionsraten der verschiedenen Metalle notiert. Das Eluat der Essigsäure/Essigsäureanhydrid-Behandlung enthielt 8 Gew.-% Methyltributylphosphonium-Salz.

Tabelle 1

| Element | Reaktionslösung vor der Behandlung mit Ionen- austauscher [Gew.-%] | Adsorptionsrate [%] | Desorptionsrate [%] |
|---------|--------------------------------------------------------------------|---------------------|---------------------|
| Rh      | 0,34                                                               | 0,0                 | –                   |
| Fe      | 0,15                                                               | 85,3                | 100                 |
| Ni      | 0,22                                                               | 62,3                | 100                 |
| Cr      | 0,07                                                               | 0,6                 | 100                 |
| Mn      | 0,01                                                               | 100,0               | 87                  |
| Mo      | 0,07                                                               | 78,0                | 5                   |

Die Adsorptionsrate wurde aus den Analysendaten rechnerisch ermittelt:

$$[\%] \text{ Adsorptionsrate} = \frac{\text{Menge Metallsalz nach Ionenaustauscher-Behandlung}}{\text{Menge Metallsalz vor Ionenaustauscher-Behandlung}} \cdot 100$$

Die Desorptionsrate wurde rechnerisch ermittelt:

$$[\%] \text{ Desorptionsrate} = \frac{\text{Menge Metallsalz vor Ionenaustauscher-Behandlung minus Menge Metallsalz nach Ionenaustauscher-Behandlung}}{\text{Menge Metallsalz im Mineralsäure-Eluat}} \cdot 100$$

Beispiel 2

1000 ml ®Lewatit SPC 112 in der H⁺-Form wurden wie im Beispiel 1 mit Essigsäure/Essigsäureanhydrid-Gemisch in einer mantelbeheizten Säule (70 mm Ø und 300 mm Länge) unter einem Druck von 1,5 bar auf eine Temperatur von 95 °C erwärmt. Bei dieser Temperatur wurden 9500 g verunreinigte und destillativ angereicherte Reaktionslösung mit einer Fließgeschwindigkeit von 80 ml/min über den Ionenaustauscher geleitet. Die Reaktionslösung bestand aus:
31,01 Gew.-% Essigsäure, 11,00 Gew.-% Essigsäureanhydrid, 30,88 Gew.-% Methyltributylphosphoniumiodid, 22,63 Gew.-% nicht bestimmte organische Phase und 4,48 Gew.-% Metallsalze.
Der beladene Ionenaustauscher wurde nach vollständigem Ablassen der Katalysatorlösung mit 7000 ml Essigsäure bei 95 °C behandelt. Das gesammelte Essigsäure-Eluat enthielt 1,2 Gew.-% Methyltributylphosphoniumsalz.
Nach der Essigsäurebehandlung wurden 2800 ml 3 molare Schwefelsäure bei 23 °C mit einer Fließgeschwindigkeit von 30 ml/min über den Ionenaustauscher geleitet. In diesem Eluat wurden die desorbierten Me- tallsalze analytisch bestimmt.

## Tabelle 2

| Element | Reaktionslösung vor der Behandlung mit Ionenaustauscher [Gew.-%] | Adsorptionsrate [%] | Desorptionsrate [%] |
|---------|------------------------------------------------------------------|---------------------|---------------------|
| Rh | 0,35 | 0,0 | - |
| Fe | 0,16 | 52,7 | 94 |
| Ni | 0,06 | 44,0 | 83 |
| Cr | 0,01 | 16,0 | 100 |
| Mn | 0,01 | 31,0 | 100 |
| Mo | 0,06 | 17,8 | 36 |

Beispiel 3

Der im Beispiel 2 regenerierte Ionenaustauscher wurde durch Behandlung mit 2000 ml Essigsäure bei 45 °C wasserfrei gespült. Mit diesem Ionenaustauscher wurde gemäß Beispiel 2 verfahren. Die Ergebnisse sind in Tabelle 3 zusammengefaßt.

## Tabelle 3

| Element | Reaktionslösung vor der Behandlung mit Ionenaustauscher [Gew.-%] | Adsorptionsrate [%] | Desorptionsrate [%] |
|---------|------------------------------------------------------------------|---------------------|---------------------|
| Rh | 0,36 | 0,0 | - |
| Fe | 0,08 | 64,7 | 99 |
| Ni | 0,04 | 48,5 | 75 |
| Cr | 0,01 | 6,7 | 100 |
| Mo | 0,05 | 6,8 | 53 |

**Patentansprüche**

1.  Verfahren zur Entfernung metallischer Korrosionsprodukte aus wasserfrei betriebenen Carbonylierungsreaktionen, bei denen Methylacetat und/oder Methanol und/oder Dimethylether an einem Edelmetallkatalysatorsystem, das aus einem Edelmetall der Gruppe VIII des Periodensystems der Elemente, einem Co-Katalysator wie Iodid oder Bromid, insbesondere Methyliodid, und einem Promotor wie Organophosphonium- oder Organoammonium-Salz sowie gegebenenfalls Lithium-Salz besteht, zu Essigsäure und/oder Essigsäureanhydrid umgesetzt werden, dadurch gekennzeichnet, daß man
    a) die mit metallischen Korrosionsprodukten verunreinigte, das Katalysatorsystem enthaltende Reaktionslösung mit einem Ionenaustauscher kontaktiert;

b) diese so kontaktierte Reaktionslösung von dem Ionenaustauscher trennt;

c) den am Ionenaustauscher adsorbierten Promotor vor einer Regenerierung mit Essigsäure und/oder Essigsäureanhydrid desorbiert;

d) das in Schritt c) erhaltene Eluat mit der in Schritt b) abgetrennten Reaktionslösung vereinigt und gemeinsam in die Carbonylierungsreaktion rückführt;

e) den aus Schritt c) erhaltenen Ionenaustauscher mit starker Mineralsäure regeneriert;

f) den so regenerierten Ionenaustauscher mit Essigsäure und/oder Essigsäureanhydrid vor dem Einsatz in Schritt a) wasserfrei wäscht.

2. Verfahren nach Anspruch 1, dadurch gekennzeichnet, daß man einen starksauren makroporösen Ionenaustauscher einsetzt, der aus einem Sulfonsäuregruppen enthaltenden Styrol/Divinylbenzol-Polymerisat besteht.

3. Verfahren nach Anspruch 1 oder 2, dadurch gekennzeichnet, daß man den Schritt a) bei Temperaturen zwischen 20 und 120 °C, insbesondere 75 und 100 °C, und Drucken von 1 bis 10 bar durchführt.

4. Verfahren nach einem der Ansprüche 1 bis 3, dadurch gekennzeichnet, daß man die verunreinigte Reaktionslösung durch ein festes Ionenaustauscherbett leitet.

5. Verfahren nach einem der Ansprüche 1 bis 3, dadurch gekennzeichnet, daß man die verunreinigte Reaktionslösung in einem Fließbett mit dem Ionenaustauscher behandelt.

6. Verfahren nach einem der Ansprüche 1 bis 3, dadurch gekennzeichnet, daß man die verunreinigte Reaktionslösung in einem Rührbehälter chargenweise mit dem Ionenaustauscher behandelt.

7. Verfahren nach einem der Ansprüche 1 bis 6, dadurch gekennzeichnet, daß man in Schritt e) als starke Mineralsäure 0,5 bis 5, insbesondere 1 bis 3 molare Jodwasserstoffsäure, Schwefelsäure oder Salzsäure einsetzt.

**Claims**

1. A process for removing metallic corrosion products from a carbonylation reaction carried out under anhydrous conditions, in which methyl acetate and/or methanol and/or dimethyl ether are reacted over a noble metal catalyst system which consists of a noble metal of group VIII of the periodic table of the elements, a co-catalyst such as iodide or bromide, in particular methyl iodide, and a promoter such as an organophosphonium or organoammonium salt, as well as, optionally, a lithium salt, to give acetic acid and/or acetic anhydride, which comprises:

a) bringing the reaction solution which is contaminated with metallic corrosion products and contains the catalyst system into contact with an ion exchanger;

b) separating this reaction solution thus brought into contact from the ion exchanger;

c) desorbing the promoter adsorbed on the ion exchanger before regeneration with acetic acid and/or acetic anhydride;

d) combining the eluate obtained in step c) with the reaction solution separated off in step b) and recycling them together into the carbonylation reaction;

e) regenerating the ion exchanger obtained from step c) with a strong mineral acid; and

f) washing the ion exchanger regenerated in this way with acetic acid and/or acetic anhydride until free from water, before use in step a).

2. The process as claimed in claim 1, wherein a strongly acid macroporous ion exchanger which comprises a styrene/divinylbenzene polymer comprising sulfonic acid groups is employed.

3. The process as claimed in claim 1 or 2, wherein step a) is carried out at a temperature between 20 and 120°C, in particular 75 and 100°C, under a pressure of 1 to 10 bar.

4. The process as claimed in one of claims 1 to 3, wherein the contaminated reaction solution is passed through a fixed ion exchanger bed.

5. The process as claimed in one of claims 1 to 3, wherein the contaminated reaction solution is treated with the ion exchanger in a fluidized bed.

6. The process as claimed in one of claims 1 to 3, wherein the contaminated reaction solution is treated with the ion exchanger batchwise in a stirred container.

7. The process as claimed in one of claims 1 to 6, wherein, in step e), 0.5 to 5, in particular 1 to 3 molar hydriodic acid, sulfuric acid or hydrochloric acid is employed as strong mineral acid.


**Revendications**

1. Procédé pour éliminer les produits de corrosion métalliques de réactions de carbonylation fonctionnant sans eau, dans lesquelles l'acétate de méthyle et/ou le métal noble et/ou l'éther diméthylique sont transformées en acide acétique et/ou en anhydride acétique sur un système catalyseur à base de métal noble qui consiste en un métal noble du groupe VIII de la classification périodique des éléments, un co-catalyseur tel qu'un iodure ou un bromure, en particulier l'iodure de méthyle, et un promoteur tel qu'un sel d'organophosphonium ou d'organoammonium ainsi éventuellement qu'un sel de lithium, caractérisé en ce que
   a) on met en contact la solution réactionnelle contenant le système catalyseur, contaminée par des produits de corrosion métalliques, avec un échangeur d'ions ;
   b) on sépare la solution réactionnelle ainsi en contact de l'échangeur d'ions;
   c) on désorbe le promoteur adsorbé sur l'échangeur d'ions avant une régénération par l'acide acétique et/ou l'anhydride acétique;
   d) on réunit l'éluat obtenu dans l'étape c) avec la solution réactionnelle séparée dans l'étape b) et on les recycle ensemble dans la réaction de carbonylation;
   e) on régénère l'échangeur d'ions obtenu dans l'étape c) par un acide minéral fort;
   f) on lave l'échangeur d'ions ainsi régénéré par l'acide acétique et/ou l'anhydride acétique en conditions anhydres avant l'utilisation dans l'étape a).

2. Procédé selon la revendication 1, caractérisé en ce que l'on utilise un échangeur d'ions macroporeux fortement acide, qui consiste en un polymère styrène/divinylbenzène à groupes acide sulfonique.

3. Procédé selon la revendication 1 ou 2, caractérisé en ce que l'on met en oeuvre l'étape a) à des températures entre 20 et 120°C, en particulier 75 et 100°C et sous des pressions de 1 à 10 bar.

4. Procédé selon l'une des revendications 1 ou 3, caractérisé en ce que l'on envoie la solution réactionnelle contaminée à travers un lit fixe d'échangeur d'ions.

5. Procédé selon l'une des revendications 1 ou 3, caractérisé en ce que l'on traite la solution réactionnelle contaminée par l'échangeur d'ions dans un lit fluide.

6. Procédé selon l'une des revendication 1 ou 3, caractérisé en ce que l'on traite la solution réactionnelle contaminée par l'échangeur d'ions en discontinu dans un récipient à agitation.

7. Procédé selon l'une des revendications 1 à 6, caractérisé en ce que l'on utilise comme acide minéral fort dans l'étape e) de l'acide iodhydrique, de l'acide sulfurique ou de l'acide chlorhydrique 0,5 à 5 M, en particulier 1 à 3 M.